# EUROPEAN PATENT APPLICATION

(11) **EP 1 302 771 A2**
(43) Date of publication of application: **16.04.2003**
(21) Application number: 02257101.2
(22) Date of filing: 14.10.2002
(51) Int. Cl.: G01N 33/84

(54) **Spin trap for use in biological systems**

(30) Priority: 15.10.2001 GB 0124711
(71) Applicant: Randox Laboratories Ltd., Crumlin, Co. Antrim BT29 4QY (GB)
(72) Inventor: Fitzgerald, Stephen Peter, Co. Antrim, BT29 4QY, N. Ireland (GB); Hamilton, Lynne, Co. Antrim, BT29 4QY, N. Ireland (GB); Winyard, Paul Graham, London EC1M 6BQ (GB)
(74) Representative: Perry, Robert Edward

(57) **Abstract**

A spin-trapping assay for nitric oxide, in a biological system such as whole blood, uses as the spin trap a lipid-soluble nitrobenzene compound such as 2,6-dimethylnitrosobenzene.

## Description

### Field of the Invention

This invention relates to the use of a spin trap in biological systems.

### Background of the Invention

It is now widely accepted that Reactive Oxygen (RO) and Reactive Nitrogen (RN) species (including free radicals) are involved in the pathogenesis of several disease states. The only technique which can detect low concentrations of radicals directly is electron spin resonance spectroscopy (ESR). Although this technique is highly sensitive (thresholds of 10⁻⁷ - 10⁻⁶ M spins), it is not directly applicable to all biological studies, as free radicals are often very short-lived and consequently may not be detected. A more useful method, permitting ESR investigation of short-lived reactive free radicals by transforming them into more persistent species, is the so-called "spin-trapping" method. The technique of spin-trapping makes use of a diamagnetic compound in which all the electrons are in pairs. The diamagnetic compound, known as the spin trap, reacts with a free radical (R^{·}) which contains an unpaired electron with spin. This reaction of the spin trap with a reactive free radical, results in the formation of a relatively stable ESR-observable spin adduct. In favourable cases, the free radical can be identified from the ESR parameters of the spin adduct (e.g. hyperfine coupling constants, g-factor).

This technique was established by Jansen *et al*, JACS (1968) 90:5909-10. Since then, there has been much research on the synthesis of suitable spin traps. The spin traps that have been most commonly employed are those designed so that, on reaction with a free radical, a nitroxide is formed.

A commonly used commercially available spin trap is 3,5-dibromo-4-nitrosobenzene sulphonate, sodium salt (DBNBS). See Kaur *et al,* JCS Chem. Comm. (1981) 142-3. DBNBS is a water-soluble spin trap which has been reported to trap superoxide, alkyl radicals and nitric oxide.

Nitric oxide (NO) has many important physiological roles. It acts as a vasorelaxant in the cardiovascular system, as a neurotransmitter in the central nervous system and it is associated with chronic inflammation. Due to increasing interest in the effects of NO, an accurate method for measurement of NO is required.

It would be desirable to detect NO in biological systems such as synovial fluid from patients with rheumatoid arthritis and plasma from patients with septic shock. However, the direct detection of NO in human biofluids is extremely difficult.

Orrell *et al,* J.C.S. Perkin Trans. 2 (1990) 1297-1303, discloses 2,6-dimethylnitrosobenzene and its monomer-dimer equilibrium. Azoulay *et al,* J.C.S. Perkin Trans. 2(1981)256-9, reports on the same characteristics, for the compound 2,4,6-trimethylnitrosobenzene.

### Summary of the Invention

The present invention is based on a realisation of the utility of lipid-soluble aromatic nitroso spin traps for the detection of nitric oxide in biological samples, as it is believed that nitric oxide in the cell is located largely in the lipid portion, and because nitric oxide is more soluble in organic solvents than aqueous systems. A lipid-soluble compound for use in the invention has the formula Ar-N=O, wherein Ar is an optionally substituted benzene ring.

### Description of the Invention

By way of example, Ar is substituted by 0 to 5 groups selected from C₁₋₆ alkyl and halogen. Typically, there are substituents at 2 and 6 positions and possibly position 4.

Preferred embodiments of compounds for use in the invention are 2,6-dibromo-4-*t*-butylnitrosobenzene (DBBNB) **(1),** 2,6-dimethylnitrosobenzene (DMNB) **(2)**, and 2,4,6-trimethylnitrosobenzene (TMNB) **(3).**

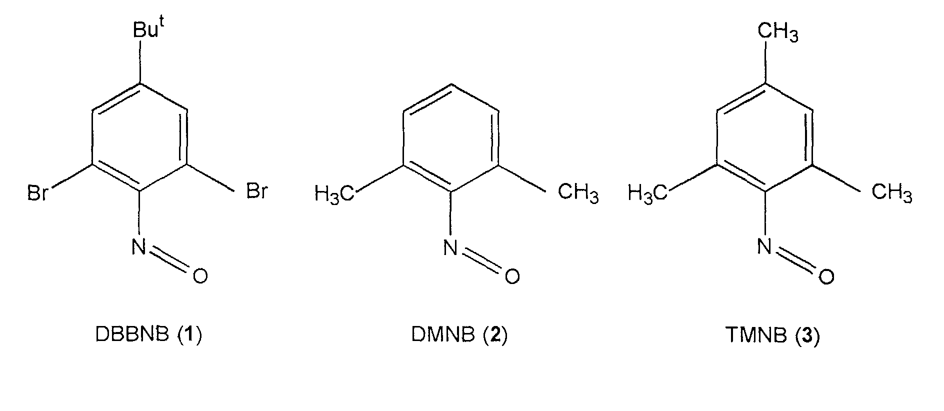

DBBNB **(1)** is a novel compound. It has now been shown to act as a spin trap for nitric oxide.

DMNB **(2)** and TMNB **(3)** are known compounds, as reported above, although their use as spin traps for nitric oxide has not been reported until now. Both DMNB **(2)** and TMNB **(3)** have now been shown to trap nitric oxide when tested in a chemical system.

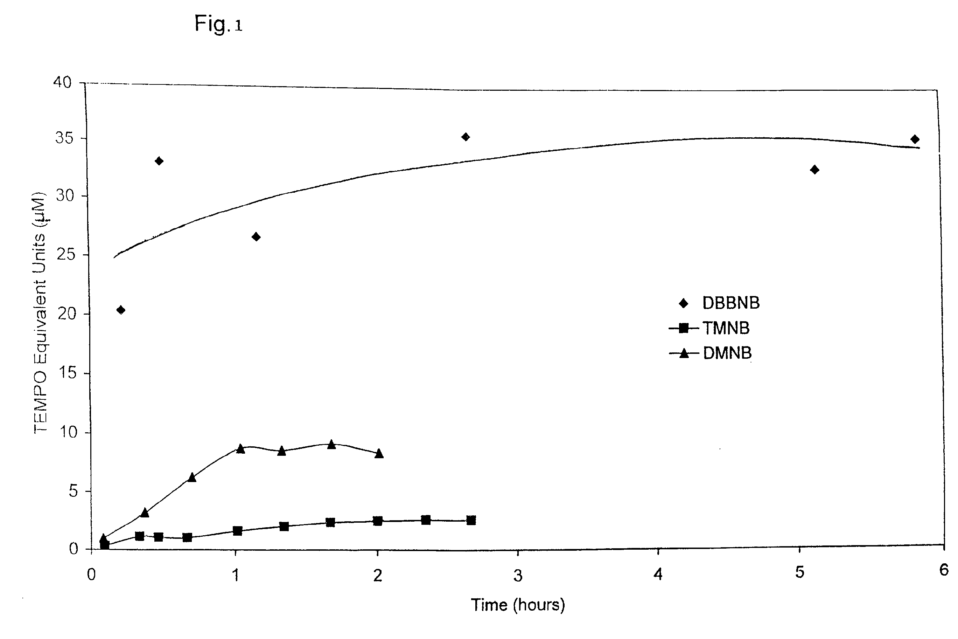

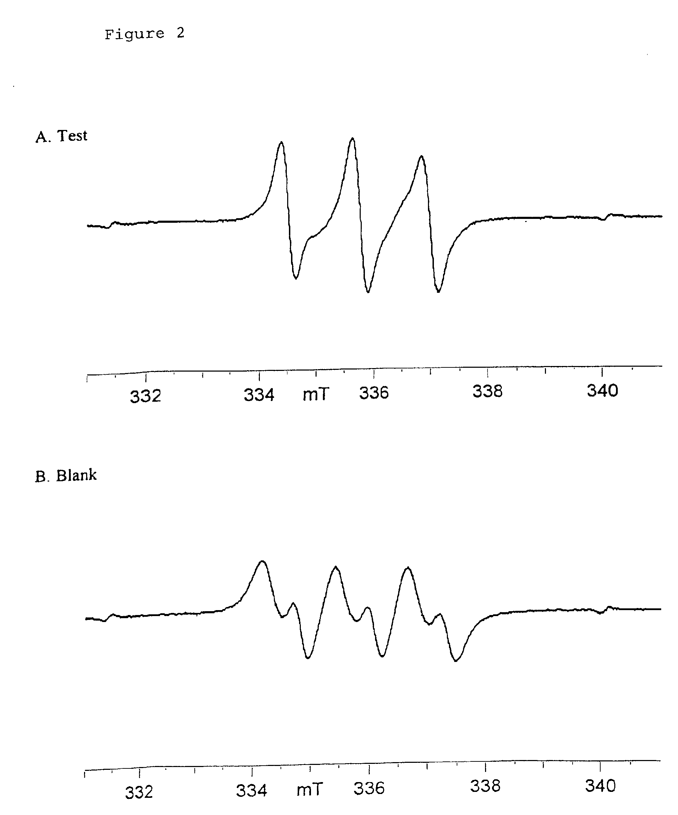

When the three lipid-soluble traps were compared, in a chemical system, the ESR signal was greatest when DBBNB **(1)** was used (Figure 1) followed by DMNB **(2),** while the smallest ESR signal was obtained for TMNB **(3).** The use of DBBNB **(1)** was accompanied by a larger background signal then for DMNB **(2)** or TMNB **(3)** (Figures 2-4). While all three tested compounds and analogues thereof are useful, DMNB **(2)** was considered to be potentially the most useful of the three lipid-soluble spin traps tested.

The biological system that is assayed may be any containing NO. Suitable systems include synovial fluid and whole blood. In cases where NO may react with thiol groups on proteins or other compounds present in the sample under test, a thiol-blocking agent may be included. Such an agent is N-ethylmaleimide.

Compounds for use in the invention are known or can be prepared by procedures generally known to those skilled in the art. Reference may also be made to the following, illustrative Examples.

### Examples

### 2,6-Dibromo-4-t-butylaniline

A solution of bromine (10.71 g; 67 mmol) in glacial acetic acid (7 ml) was added dropwise to a solution of 4-*t*-butylaniline (5.0 g; 33.5 mmol), which was cooled in an ice bath. When addition was complete, the reaction mixture was stirred at room temperature for 1 hr. The yellow/orange suspension was poured into water (200 ml) to give two layers. Methylene chloride (50 ml) was added and the organic phase was isolated. The organic phase was washed with water until the washings were pH = 7 (3 x 50 ml). The organic phase was dried (Na₂SO₄), filtered and the solvent removed under vacuum to give the crude product as a pale orange oil. Crude yield 8.0 g. Purification by column chromatography on silica gel (eluant 5% ethyl acetate/hexane) gave the pure product as an oil. Yield 7.4 g (75%). T.l.c. on silica gel (10% ethyl acetate/hexane), Rf = 0.58.

### I.R. Observed 733 cm⁻¹ (s) C-Br.

### 2,6-Dibromo-4-t-butylnitrosobenzene (DBBNB) (1)

A solution of 2,6-dibromo-4-*t*-butylaniline (5.0 g; 15.5 mmol) in anhydrous methylene chloride (25 ml) was added dropwise to a solution of m-chloroperoxybenzoic acid (72% purity) (7.45 g; 31 mmol) in anhydrous methylene chloride (250 ml) in an ice bath. The reaction mixture was stirred at 0°C for 2hr and then at room temperature overnight. It was then washed with aqueous sodium carbonate (20%) (4 x 150 ml) and water (1 x 150 ml). The organic phase was dried (Na₂SO₄), filtered and the solvent removed under vacuum to give the crude product as a yellow solid. Crude yield 5.2 g. The product was recrystallised from methanol to give a yellow crystalline solid. Yield 4.0 g (77%).
Note: Upon melting or in solution the compound goes from a yellow crystalline solid (dimer) to a bright green liquid (monomer). This confirms the product is a nitroso compound.
M.p. 106-108°C (from methanol).
I.R. 1290 cm⁻¹ (s) (aromatic C-nitroso *trans* dimer).
M.S. (+ E.I.) Peaks observed at 319, 321, 323 (51%, 100%, 52%) (M⁺). Peaks expected at 319, 321, 323 (1:2:1) (M⁺).

### 2,6-Dimethylnitrosobenzene (DMNB) (2)

A solution of 2,6-dimethylaniline (6.0 g; 49.5 mmol) in anhydrous methylene chloride (75 ml) was added dropwise to a solution of *m*-chloroperoxybenzoic acid (72% purity) (18.6 g; 99 mmol) in anhydrous methylene chloride (640 ml) in an ice bath. The reaction mixture was stirred at 0°C for 2hr and then at room temperature overnight. The solvent was reduced in volume to approximately 400 ml, then washed with aqueous sodium carbonate (20%) (4 x 150 ml) and water (1 x 150 ml), dried (Na₂SO₄) and the solvent was removed under reduced pressure to give the product as a yellow solid, crude yield 6.5 g. This was recrystallised from methanol to give a pale yellow crystalline solid. Yield 3.1 g (46%).
M.p. 131-134°C (from methanol).
I.R. 1255cm⁻¹ (s) (aromatic C-nitroso *trans* dimer). Literature states 1253-1299 cm⁻¹ for aromatic C-nitroso *trans* dimer.
M.S. (+ E.I.) Peak observed at 135 (M⁺, 100%).

### 2,4,6-Trimethylnitrosobenzene (TMNB) (3)

A solution of 2,4,6-trimethylaniline (6.0 g; 44.4 mmol) in anhydrous methylene chloride (75 ml) was added dropwise to a solution of m-chloroperoxybenzoic acid (72% purity) (21.3 g; 88.8 mmol) in anhydrous methylene chloride (640 ml) in an ice bath. The reaction mixture was stirred at 0°C for 2 hr and then at room temperature overnight. The solvent was reduced in volume to approximately 400 ml, then washed with aqueous sodium carbonate (20%) (4 x 150 ml) and water (1 x 150 ml), dried (Na₂SO₄) and the solvent removed under reduced pressure to give the crude product. This was recrystallised from methanol to give the product as a pale yellow solid. Yield 3.5 g (53%).
M.p. 120-122°C (from methanol).
I.R. 1263 cm⁻¹ (s) (aromatic nitroso *trans* dimer).
M.S. (+ E.I.) Peak observed at 149 (M⁺, 100%).

### ESR analysis

ESR spectra were obtained using a prototype spectrometer (Jeol (U.K.) Ltd., Welwyn Garden City, England) equipped with a TE₀₁₁ cylindrical cavity. Samples were analysed at room temperature in a WG-LC-11 quartz flat cell (Wilmad-Glass, Buena, NJ). In general, the instrument parameters were: microwave frequency 9.2 GHz, microwave power 4 mW, centre field (CF) 336.7 mT, sweep width (SW) ± 5 mT, number of data points 4095, modulation frequency 100 kHz. For the spin traps reacted with nitric oxide: sweep time (ST) 150 s, time constraint (TC) 0.3 s, modulation width (MW) 0.2 mT. A JEOL ES-DM1 digital manganese oxide marker (0.03 mm of the glass tube inserted per unit on the dial setting) with a dial setting of 500 was used to ensure reproducibility between samples and to identify the position of the signals in the microwave field.

### Spin-trapping solutions

Samples of DBBNB, DMNB and TMNB were weighed into vacutainers, which were then evacuated. Toluene and Tris-HCI buffer, 0.01 M, pH 7.4, were deoxygenated by bubbling with nitrogen gas for 15 min. Deoxygenated toluene was added to DBBNB, DMNB and TMNB, using a gas-tight syringe, to give a final concentration of 0.15 M in the reaction mixture.

### Reaction of nitric oxide with spin traps

### NO-saturated water

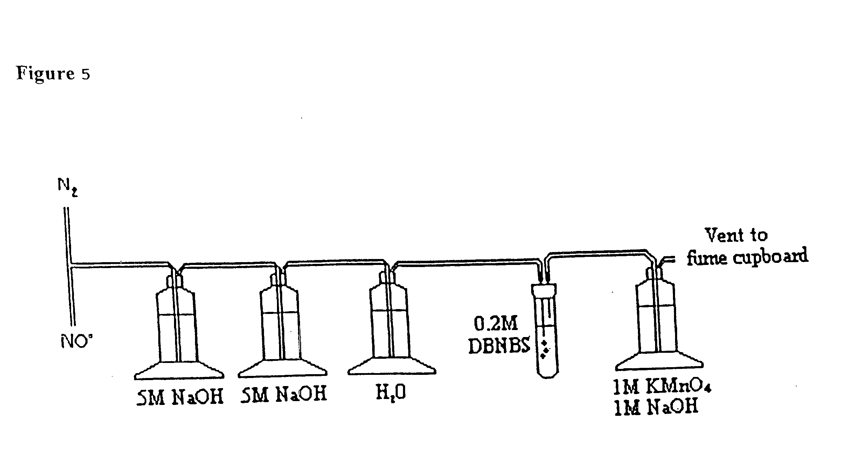

The apparatus shown in Figure 5 was flushed with nitrogen for 15 min. The vacutainer containing deionised water (4 ml) was then connected to the system and flushed with nitrogen for a further 15 min, to remove any oxygen present in the system, and therefore to prevent the formation of nitrogen dioxide. The NO gas was bubbled through the deionised water *via* the system for 45 min. The small amount of nitrogen dioxide present in the pressurised NO container was removed by bubbling through two bottles of 5M NaOH. The gas was then bubbled through a scrubbing bottle containing deionised water to remove any alkaline aerosol contamination. Any excess of NO after the deionised water step was bubbled through a scrubbing bottle containing 1M potassium permanganate/1M NaOH to prevent excess NO escaping from the fumehood. The concentration of NO in the resulting NO-saturated water was taken to be 2.0 mM.

### Time Course

NO-saturated water (1 ml) was added to 1 ml of the spin trap solutions of DBBNB, DMNB and TMNB. The reaction mixture was then incubated at room temperature. The final concentration of the spin traps was 0.15 M and the final concentration of NO was 1 mM. Parallel blanks were carried out by adding 1 ml of deoxygenated water to the spin traps. All the reaction mixtures were incubated at room temperature. At each time point samples were taken from both test and blank tubes for ESR spectroscopic analysis and the blank was subtracted from the spectra. Results for the time course experiments are shown in Figure 1.

### Sensitivity study of DMNB reacting with nitric oxide

The NO-saturated water, was diluted with deoxygenated water to give final concentrations in the reaction mixture of 0, 0.25, 0.5, 1, 5, 10, 50, 100, 200 and 400 µM. The NO solutions (0.5 ml) were then added in turn to 0.5 ml aliquots of spin trap solutions. The final spin trap concentration was 0.1 M throughout. The resulting mixtures (10 samples) were shaken for 1 min and incubated on a roller for 1 hr 40 min at room temperature. The signal to noise ratio was calculated from each spectrum and the blank was obtained by adding 0.5 ml of deoxygenated water to the spin trap.

### Reaction of nitric oxide with DBBNB, DMNB and TMNB

All three lipid-soluble spin traps were shown to trap nitric oxide. The ESR signal height was greatest when DBBNB **(1)** was used, followed by DMNB **(2)** and then TMNB **(3)** (Figure 1).

When DBBNB **(2)** was tested, the resulting DBBNB-NO adduct gave rise to a broad three-line signal with a hyperfine coupling constant of a_{N} = 1.25 mT, g value = 2.0072 and peak width = 0.728 mT, at low resolution (MW 0.2 mT) (Figure 2A). However, the blank control of DBBNB alone gave rise to a six-line signal, which did not correspond to the three-line signal obtained in the test sample (Figure 2B). The ratio of the signal intensity in the test sample to that in the blank varied from 3:1 to 1:1, depending on the DBBNB concentration and incubation time. This suggests that DBBNB (**1**) may be a good spin trap for nitric oxide in biological systems once reduction/elimination of the background signal is achieved.

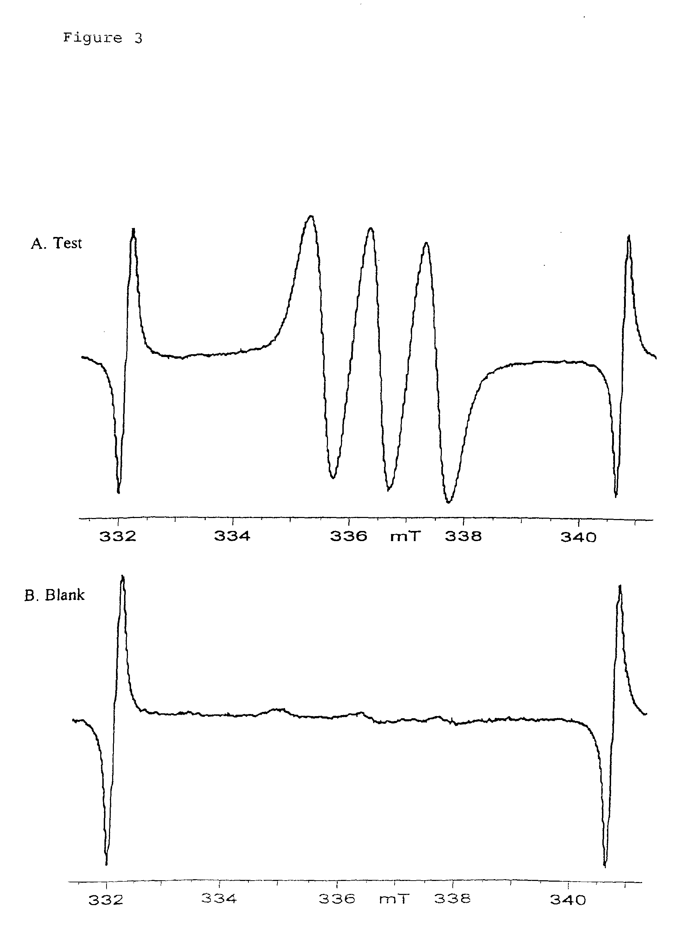

When DMNB **(2)** was tested, the DMNB-NO adduct also gave a broad three-line signal with a hyperfine coupling constant of aN = 1.07 mT, g value = 2.0073 and peak width = 0.624 mT (Figure 3A). Very little background signal was observed in the blank (Figure 3B). High resolution scanning revealed no further splitting. This indicates that DMNB may be an excellent lipid-soluble spin trap for nitric oxide in biological systems.

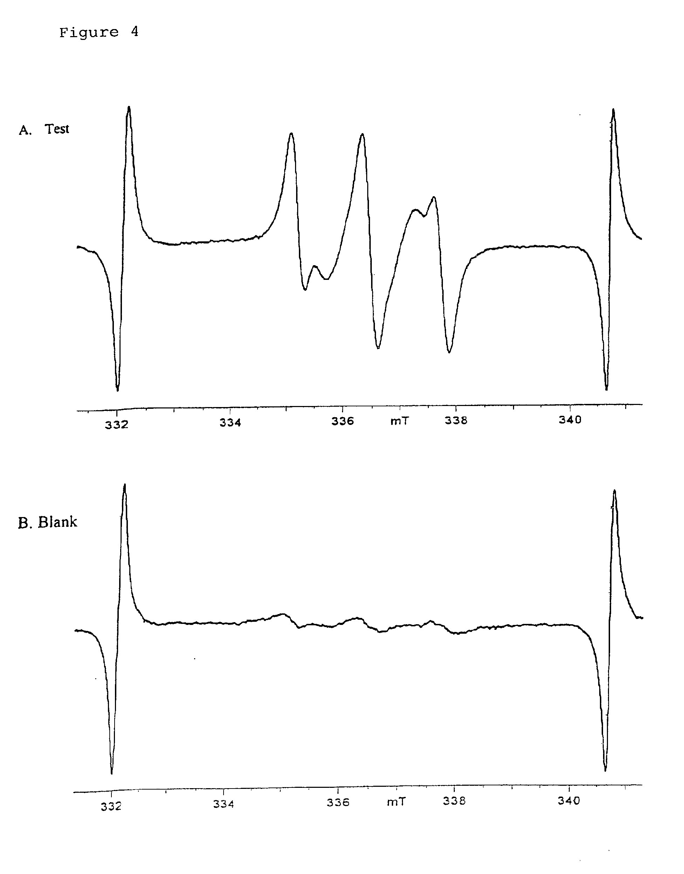

When TMNB **(3)** was tested as a spin trap for nitric oxide, the TMNB-NO adduct gave a more complex spectrum (Figure 4A) consisting of a three-line signal and additional lines, which were not resolved from the three-line signal. The three-line signal had a hyperfine coupling constant of a_{N} 1.27 mT, g value 2.0069 and peak width = 0.624 mT. The extra lines may have been produced by the methyl group at the para position with respect to the nitroso group. These extra lines suggest that TMNB may be less preferred than other compounds for use in the invention, in the determination of signal intensity or signal area.

### Time course for DBBNB, DMNB and TMNB reacting with nitric oxide

A time-course study revealed that the reaction of DBBNB **(1)** with nitric oxide reached completion at approximately 5 hr, the reaction of DMNB **(2)** with nitric oxide reached completion at approximately 1 hr 40 min and the reaction of TMNB **(3)** with nitric oxide reached completion at approximately 2hr 40 min (Figure 1). All three reaction times are a significant improvement on the reaction time of DBNBS with NO of approximately 50 hr. When all the results are taken into consideration, DMNB may be the best of the three lipid-soluble traps for nitric oxide.

### Sensitivity of DMNB reacting with nitric oxide

The limits of detection and quantitation for DMNB with nitric oxide are given in the table below.

| | Limit of detection (µM) (S/N = 3) | Limit of quantitation (S/N = 10) |
|---|---|---|
| DMNB | 6.73 | 79.57 |

DMNB is potentially a very useful spin trap for nitric oxide in biological matrices where the lipid solubility of the spin trap should improve performance The increased reaction time of DMNB with nitric oxide should also be advantageous.

### Spin trapping of nitric oxide in whole blood using DMNB (2)

Initial experiments suggested that peripheral blood incubated with DMNB **(2)** in toluene did not produce a detectable DMNB-NO adduct signal. This may be due to the possibility that any NO present or produced by live leucocytes in whole blood may react with thiol groups to form S-nitrosothiols and consequently be not trapped by DMNB. Hence N-ethylmaleimide (NEM), which is a blocking reagent for thiols, was used to block the thiol group in plasma proteins to stop them reacting with NO, thereby increasing the possibility of formation of the spin trap adduct DMNB-NO. Lipopolysaccharide (LPS), which activates cell function, was also used in order to stimulate the cells to increase production of NO. As NEM may be toxic to cells, it was added i) at the same time as LPS and ii) 3.5 hours after the addition of LPS.

Peripheral blood was taken from a healthy volunteer into a vacutainer containing EDTA. After mixing, 1 ml of the blood was dispensed into each reaction vacutainer by a syringe, as quickly as possible (under 5 min).

DMNB **(2)** 0.5406 g was dissolved in 16 ml of deoxygenated toluene (by bubbling through nitrogen gas) to make a 0.25 M solution. 700 µl of the solution was added to each reaction mixture to make a final concentration of 0.1 M. A NO solution was made by bubbling nitric oxide gas through deoxygenated water for 45 min. The resulting NO solution (2 mM) was diluted with deoxygenated water to give 10, 35 and 50 nM solutions. An NEM solution (0.3M) was prepared by dissolving 0.0751 g of NEM in deoxygenated water. NEM solution (0.3 M), in amounts of 20, 40 and 80 µl, was added to the reaction mixtures to give a concentration, in 1 ml of blood, of 6, 12 and 24 mM. LPS (1 mg) was dissolved in 1 ml of PBS, 0.01 M, pH 7.4. The working stock solution is 100 µg/ml, diluted with the same buffer. The working stock solution of 10 µl was added to the reaction mixture to give a 1 µg/ml of blood. All transfer of reagents was done by gastight syringe through rubber stopper. The difference in volume due to the addition of NEM and LPS was compensated for by addition of deoxygenated water.

NO solution, blood without NEM nor LPS and PBS (0.01 M, pH 7.4) as controls were added to DMNB in toluene directly. NEM without LPS was added to blood immediately prior to addition of DMNB in toluene. When LPS was added to blood alone, the mixture of blood and LPS was incubated at room temperature for 3.5 hour before addition to DMNB in toluene. When both NEM and LPS were added to blood, NEM was either added to the blood together with LPS or added 3.5 hour later (just before addition of DMNB). When the DMNB in toluene was added, the reaction mixtures were incubated at room temperature on a roller for 2 hour to ensure effective extraction.

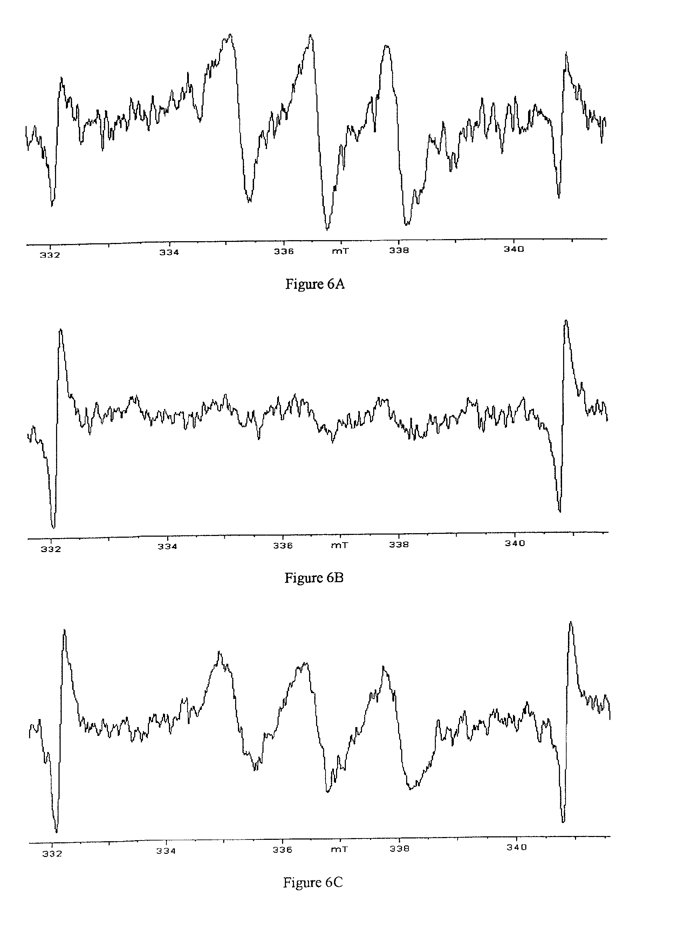

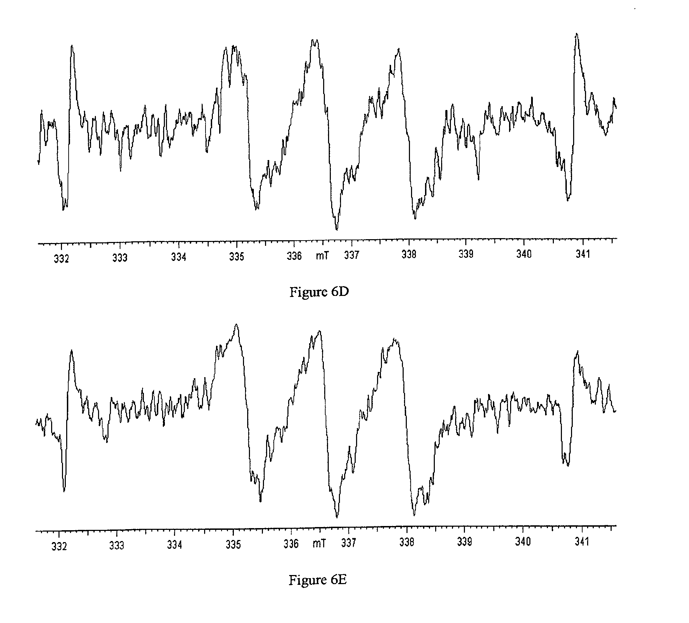

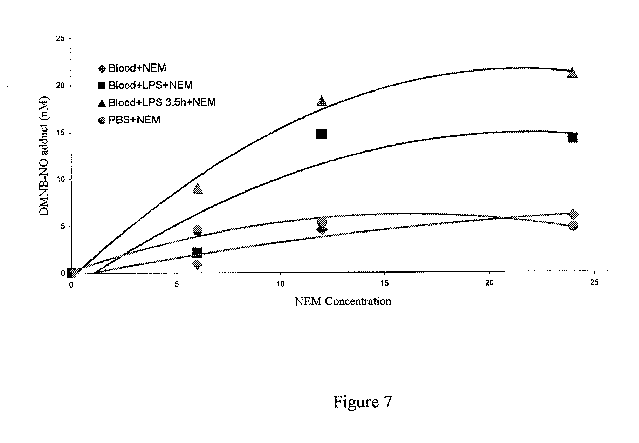

The reaction mixture was then analysed by ESR spectrometry (RE1X, Jeol). Individual results are shown in Figure 6, and have been reassessed, for comparison purposes, in Figure 7. In Figure 6, the respective spectra are for:
A) NO (10 nM) incubated with DMNB (0.1 M) in toluene
B) Whole blood (1 ml) incubated with DMNB (0.1 M) in toluene
C) Whole blood (1 ml) plus NEM (12 mM) incubated with DMNB (0.1 M) in toluene
D) Whole blood (1 ml) incubated with LPS (1 µg) and NEM (12 mM) for 3.5 hours, then incubated with DMNB (0.1 M) in toluene
E) Whole blood (1 ml) incubated with LPS (1 µg) for 3.5 hours, then incubated with DMNB (0.1 M) in toluene after addition of NEM (12 mM)

When peripheral blood was incubated with DMNB **(2)** in toluene, very little signal was detected. Adding NEM to the blood before incubating with DMNB **(2)** resulted in an increase in signal intensity. The increase was proportional to NEM concentration, indicating that a higher concentration of NEM resulted in increased blocking of thiol groups in the plasma, thus increasing the chance for DMNB trapping of NO (produced by live leucocytes in the blood). Addition of LPS alone did not increase the signal intensity, as the NO produced could react with unblocked thiol groups. However, addition of both LPS and NEM caused a significant increase in the DMNB-NO signal. Addition of NEM after incubating LPS with blood for 3.5h and then reaction with DMNB produced an even larger signal. This may be due to the cell toxicity of NEM, as it may bind the thiol groups in the cell membrane protein. Therefore, prolonged incubation of NEM with blood should be avoided.

The concentration of NO may be quantified by construction of a relevant standard curve.

## Claims

1. An assay for nitric oxide in a biological system, using a spin trap, wherein the spin trap is a lipid-soluble nitrosobenzene compound.

2. An assay according to claim 1, wherein the spin trap is 2,6-dibromo-4-tert-butylnitrosobenzene.

3. An assay according to claim 1, wherein the spin trap is 2,6-dimethylnitrosobenzene.

4. An assay according to claim 1, wherein the spin trap is 2,4,6-trimethylnitrosobenzene.

5. An assay according to any preceding claim, wherein the biological system is a sample of whole blood.

6. An assay according to claim 5, which additionally comprises using an agent that blocks the reaction of nitric oxide with thiols.
